# EUROPEAN PATENT APPLICATION

(11) **EP 4 218 830 A1**
(43) Date of publication of application: **02.08.2023**
(21) Application number: 23153625.1
(22) Date of filing: 27.01.2023
(51) Int. Cl.: A61K 49/00

(54) **TUMOR TARGETED DIAGNOSTIC IMAGING AGENT FOR DIAGNOSTIC BIOPSY, OR INTRAOPERATIVE TUMOR IDENTIFICATION OR MARGIN ASSESSMENT USING NEAR-INFRARED FLUORESCENCE (NIRF) IMAGING**

(30) Priority: 27.01.2022 KR 20220012005
(71) Applicant: NanoMedi Pharm. Co., Ltd., Daejeon 34520 (KR)
(72) Inventor: OH, Keun Sang, 34605 Daejeon (KR)
(74) Representative: Schiweck Weinzierl Koch Patentanwälte Partnerschaft mbB

(57) **Abstract**

The present invention provides a technique for preparing a nanoplatform-based tumor-targeted diagnostic contrast agent which enables precise local diagnosis of cancer lesions, and the nanoplatform-based tumor-targeted diagnostic contrast agent can avoid the situation in which normal tissue is unnecessarily excised or tumor tissue is not excised when tumor removal surgery is performed under fluorescence image guidance, and can be visualized by emitting light under near-infrared ray induction as the contrast agent is directly applied to the local area and then quickly and accurately penetrates/is absorbed into the cancer lesion so that a clinical surgeon efficiently and conveniently performs surgery in an operating room during laparoscopic surgery with minimal incision or open surgery according to the occurrence site and progression of cancer.

## Description

### CROSS-REFERENCE TO RELATED APPLICATION

This application claims priority to and the benefit of Korean Patent Application No. 2022-0012005, filed on January 27, 2022, the disclosure of which is incorporated herein by reference in its entirety.

### BACKGROUND

### 1. Field of the Invention

The present invention relates to a tumor-targeted diagnostic contrast agent for tumor identification and resection margin separation during a diagnostic biopsy or tumor removal surgery using near-infrared fluorescence (NIRF) imaging.

### 2. Discussion of Related Art

Advances in diagnosis and treatment using regular cancer screening and precision medicine have led to an increase in early detection of cancer, and thus the survival rate for cancer is increasing, but cancer is still very frightening and menacing to human health.

Cancer is mainly treated through surgical resection, and the currently conducted surgical resection directly removes cancer through extensive resection to clearly remove the cancer site developed in the form of tissue. Although this is effective for treatment, when some of the surrounding tissue containing cancer cells remains, metastasis of cancer cells occurs after surgery, and when an excessively wide area is removed, there is a concern about degradation of organ function. Therefore, recently, a surgery method for cancer patients is being developed from extensive resection (total resection) to function-preserving resection. Also, there is a need for precise and effective cancer surgery with minimal resection, which minimizes complications while completely excising cancer tissue in consideration of the cancer progression and health condition of a patient diagnosed with cancer.

Meanwhile, with the development of medical equipment, laparoscopic surgery called "minimally invasive surgery," which uses special instruments such as laser or forceps by making a small hole without incision of the abdomen and then inserting a laparoscope equipped with a special camera, has recently been frequently performed for the purpose of compensating for the disadvantages of traditional open surgery, such as a large incision and scar and heavy bleeding, and a long recovery time, because a large incision is made in the abdomen. However, the laparoscopic surgery still relies on a surgeon's visual and tactile senses and the experience gained through numerous operations to identify the location of cancer and distinguish its boundary.

Although medical contrast agents and diagnostic methods such as radiography, computed tomography (CT), magnetic resonance imaging (MRI), and positron emission tomography (PET) are used for anatomically and functionally accurate diagnosis before surgery in clinical practice, these methods do not accurately reflect most of the movement of a patient in real time. Therefore, there is a disadvantage in that the boundary between the location of cancer and normal tissue is not accurately identified in the living organ of the human body during an actual operation after imaging, and the identification is more difficult when there is inflammation or anatomical variation or when anticancer therapy and radiotherapy are performed before surgery.

In order to overcome the limitations, attempts using ultrasound images have been made, but there is still a problem in that it is difficult to accurately determine the three-dimensional location of the tumor boundary through ultrasound analysis passively performed by a human hand. In addition, a method, in which a radioactive substance such as technetium (Tc) is injected into the vicinity of a cancer lesion using an endoscope during an operation on an anesthetized cancer patient, metastasis in a sentinel lymph node, which is the first lymph node to which cancer cells are spread from a primary tumor through a lymphatic vessel, is identified using equipment such as a "gamma probe" through a biopsy during surgery, and then the scope of surgery is determined, is used. However, this method has also a disadvantage in that there is a risk of radiation overexposure to patients and medical personnel, and direct identification is not made through a surgeon's visual and tactile senses during an operation, but made through sound or numerical values using a probe called a "gamma probe." Also, the method has a disadvantage in that a surgeon needs a long learning and adaptation period for complex medical equipment technology, and still has a limitation in that radiopharmaceuticals discharged from a patient's body administered for diagnosis/treatment may be released into the general environment and cause social problems such as radiophobia.

Therefore, when a surgeon can view tumor images in real time during an operation using fluorescence imaging equipment after injecting a fluorescent dye (contrast substance) capable of targeting a tumor or applying the fluorescent dye to lesions by direct spraying in a tumorectomy, this method will be able to be used as a method that may be selected to solve the limitations of conventional methods. Also, the method is expected to minimize complications and allow patients to recover quickly.

Representative examples of the fluorescent substance used in the field of research and development include organic contrast substances and inorganic quantum dots (QDs) that are semiconductor constituents and spontaneously emit light. The inorganic quantum dots are applied to bioimaging, immunology, and a molecular diagnosis kit due to having unique and various optical properties. However, since the inner core thereof is composed of a toxic substance, there is still a problem of safety in the human body, so the inorganic quantum dots have been used only in animal experiments and not applied in clinical practice. Indocyanine green (ICG) and methylene blue (MB), which are mainly used as organic contrast substances, have already been clinically used in liver function evaluation, fluorescein angiography (indocyanine green angiography), ICG near-infrared cholangiography, fluorescent lymphography, and the like.

When a near-infrared fluorescence medical device and a fluorescent diagnostic contrast agent are used in combination, a surgeon can easily view tumor images through visual observation in real time in an operating room, and the fluorescent diagnostic contrast agent administered on the spot according to a medical field condition can be tracked in real time, thereby making it possible to identify the travel route of metastatic lymphatic vessels and even lymph nodes.

Fluorescence-guided surgery is a method that provides information about the location and shape of tumors to a surgeon in real time and is mainly focused on diagnosis. As an optimal clinical auxiliary diagnostic technology is developed while performing surgical removal of primary tumors and lymph nodes suspected of metastasis in patients including colorectal cancer and breast cancer patients, it is judged that it will be able to dominate the market related to precision cancer surgery based on an advanced concept source technology for accurate judgment of patients suffering from cancer and clinicians performing on-site surgery.

In addition, the technology can replace imported products whose supply and demand are domestically unstable, can allow additional research to be activated in fields related to types of cancer in addition to breast cancer and robot surgery, laparoscopic surgery, and endoscopic surgery in addition to general surgery as follow-up research, and can be clinically applied to minimally invasive surgery, and therefore, the development potential thereof is high. When the technology is established as a standard surgical method that can provide dramatic help in a range of surgeries from open surgery to laparoscopic surgery, it is expected to attract considerable interest as an effective cancer removal surgery method in medical communities around the world, inspire the development of nanofusion-based formulation technology which is currently very stagnant, and help activate source technology development and related research.

### [Related Art Documents]

### [Patent Documents]

1. Korean Registered Patent No. 10-0035407
2. Korean Registered Patent No. 10-0029745
3. Korean Registered Patent No. 10- 0070047
4. Korean Registered Patent No. 10- 0064971

### SUMMARY OF THE INVENTION

The present invention is directed to providing a technique for preparing a nanoplatform-based tumor-targeted diagnostic contrast agent which enables precise local diagnosis of cancer lesions, and the nanoplatform-based tumor-targeted diagnostic contrast agent can avoid the situation in which normal tissue is unnecessarily excised or tumor tissue is not excised when tumor removal surgery is performed under fluorescence image guidance, and can be visualized by emitting light under near-infrared ray induction as the contrast agent is directly applied to the local area and then quickly and accurately penetrates/is absorbed into the cancer lesion so that a clinical surgeon efficiently and conveniently performs surgery in an operating room during laparoscopic surgery with minimal incision or open surgery according to the occurrence site and progression of cancer.

One aspect of the present invention provides a method of preparing a nano-DDS-based tumor-targeted diagnostic contrast agent, which includes: mixing a polysorbate, soybean oil, and a staining substance to form a first mixture; mixing the first mixture with a poloxamer and a stabilizer and heating the resulting mixture at 40 to 70 °C to form a second mixture; and cooling the second mixture to form a nanoplatform solid, wherein the staining substance is a staining substance that emits light at a wavelength of 650 to 800 nm, and the stabilizer is one or more selected from the group consisting of Solutol and vitamin E TPGS.

Another aspect of the present invention provides a nano-DDS-based tumor-targeted diagnostic contrast agent which is a nanocomposite with a micelle structure, wherein an inner core of the micelle structure includes soybean oil and a polysorbate in which a staining substance is dissolved, and an outer shell of the micelle structure includes a poloxamer and a stabilizer.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:

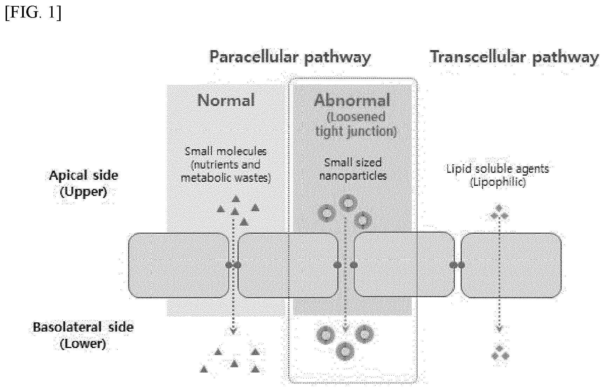

FIG 1 is a schematic diagram showing the absorption/penetration mechanism of a nano-DDS-based tumor-targeted diagnostic contrast agent according to one aspect of the present invention into tissue;

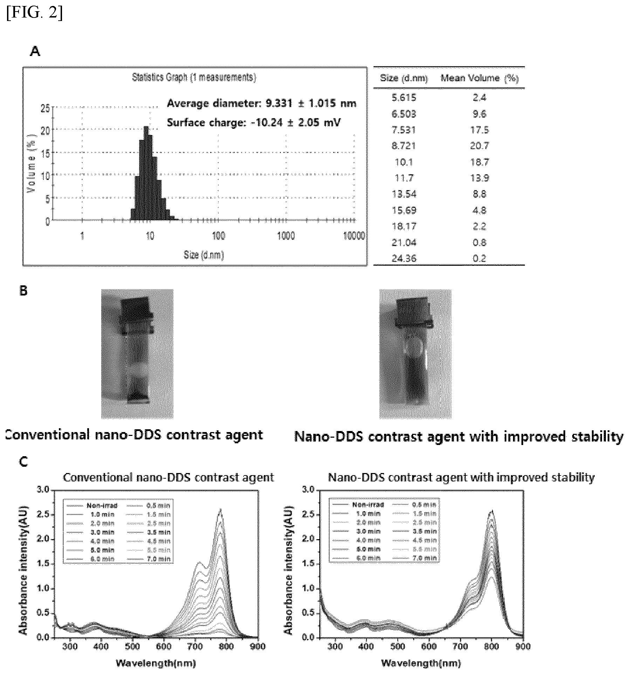

FIG 2A shows the particle distribution of a nano-DDS-based tumor-targeted diagnostic contrast agent prepared in an example of the present invention in an aqueous solution, FIG 2B shows a change of the diagnostic contrast agent in an aqueous solution, and FIG 2C shows light stability by an external light source;

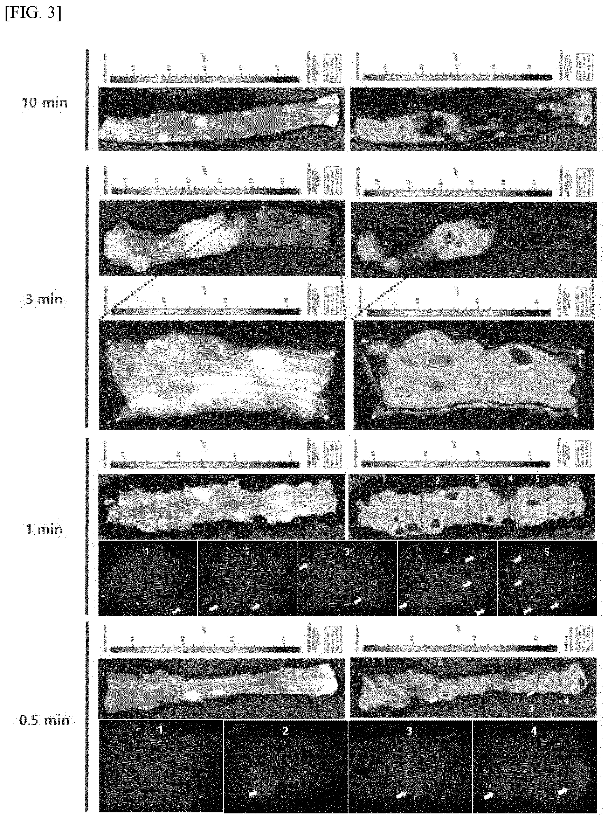

FIG 3 shows the results of observing a colorectal cancer-induced mouse model over time using real-time near-infrared fluorescence diagnostic equipment for small animals after directly spraying a nano-DDS-based tumor-targeted diagnostic contrast agent prepared in an example of the present invention onto the mouse model and performing washing;

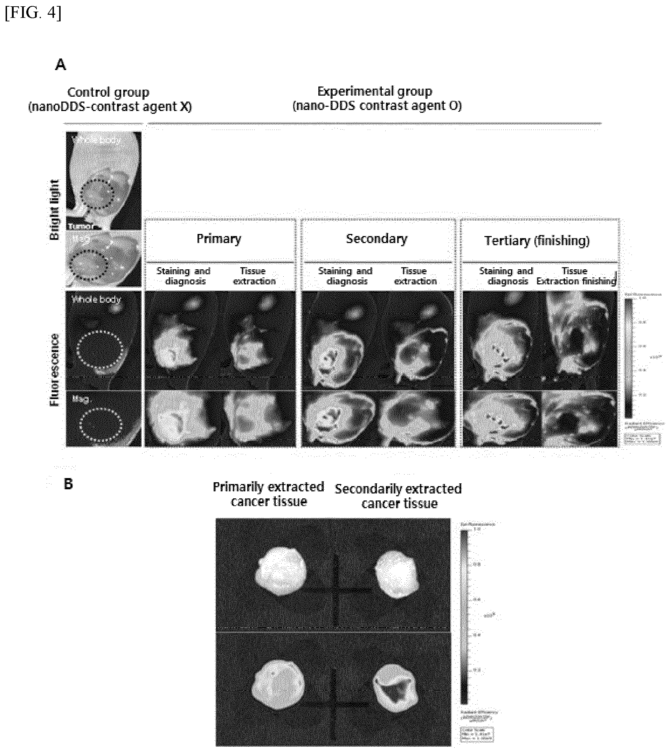

FIG 4 shows images obtained by measurement according to a repeated process using near-infrared fluorescence imaging equipment after a process of spraying a nano-DDS-based tumor-targeted diagnostic contrast agent prepared in an example of the present invention onto the multiple tumors of a tumor-induced mouse model, performing diagnosis, and extracting cancer tissue is repeatedly performed;

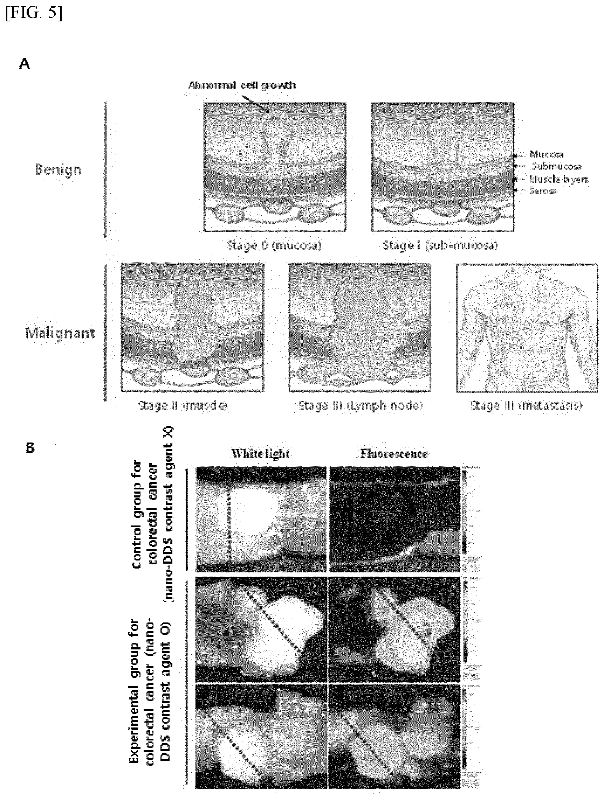

FIG 5A is a schematic diagram showing various histological stages of the progression of colorectal cancer, and FIG 5B shows images obtained by measurement using near-infrared fluorescence imaging equipment after a nano-DDS-based tumor-targeted diagnostic contrast agent prepared in an example of the present invention was directly sprayed onto the multiple tumors of an inflammatory colorectal cancer-induced mouse model and washed;

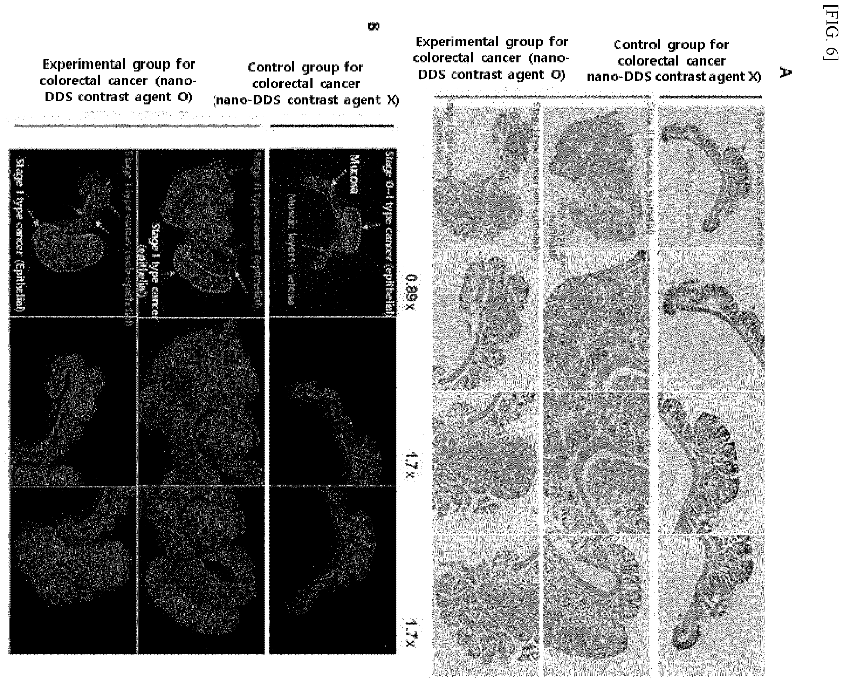

FIGS. 6A and 6B show images obtained by measuring the tissue stained with H&E using a microscope and images obtained by measuring the same tissue section using a fluorescence microscope, respectively, after a nano-DDS-based tumor-targeted diagnostic contrast agent prepared in an example of the present invention was directly sprayed onto the multiple tumors of an inflammatory colorectal cancer-induced mouse model and washed, and the tissue whose cancer diagnosis was determined was sliced into the form of a section; and

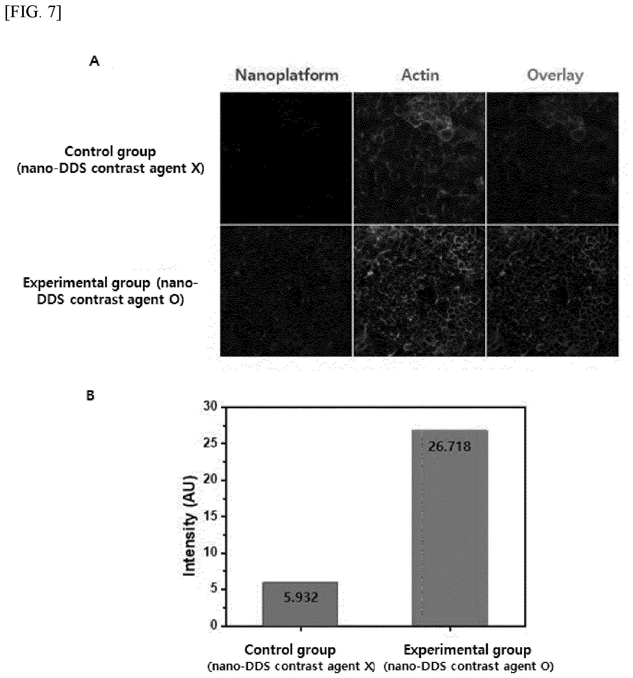

FIG 7 shows images and fluorescence quantitative analysis results obtained by measurement using a fluorescence microscope after a nano-DDS-based tumor-targeted diagnostic contrast agent prepared in an example of the present invention was applied to three-dimensionally cultured cells and washed to confirm the penetration/absorption mechanism into cancer tissue.

### DETAILED DESCRIPTION OF EXEMPLARY EMBODIMENTS

Hereinafter, the present invention will be described in detail.

While the present invention may be modified in various ways and take on various alternative forms, the following specific embodiments and descriptions disclosed herein are provided to aid understanding the present invention, and are not intended to limit the present invention to specific embodiments. It should be understood that the scope of the present invention includes all changes, equivalents, or substitutes which are included within the spirit and technical scope of the present invention.

As used herein, the terms "first" and "second" do not mean an order, and since they are used to distinguish two components, they do not limit the two components. In particular, each of a "first mixture," a "second mixture," and a "third mixture" refers to a mixture including mixing elements presented before each term appears, and the terms "first," "second," and "third" are used to distinguish three mixtures.

The present invention relates to a method of preparing a nano-DDS-based tumor-targeted diagnostic contrast agent, which includes:
mixing a polysorbate, soybean oil, and a staining substance to form a first mixture (hereinafter, referred to as a first mixture formation step);
mixing the first mixture with a poloxamer and a stabilizer and heating the resulting mixture at 40 to 70 °C to form a second mixture (hereinafter, referred to as a second mixture formation step); and
cooling the second mixture to form a nanoplatform solid (hereinafter, referred to as a nanoplatform solid formation step).

In the present invention, a "nano-drug delivery system (nano-DDS)" refers to a nanotechnology-based drug delivery system and is a technology that delivers a drug to a desired body part using an agent or releases the drug at an appropriate time. In other words, the purpose of the nano-DDS is to maximize therapeutic efficacy and effects by selectively delivering a drug to a target site and optimizing an effective blood concentration for a long time according to a disease and to minimize side effects of the drug. In the present invention, the nano-DDS may be expressed as a DDS-based nanoplatform.

In the present invention, a "nano-DDS-based tumor-targeted diagnostic contrast agent" refers to a nanoplatform loaded with a staining substance for cancer diagnosis, and the nanoplatform may be used as a contrast agent. Specifically, the nano-DDS-based tumor-targeted diagnostic contrast agent is a nanocomposite with a micelle structure, and a staining substance may be loaded in an inner core of the micelle structure.

In the present invention, the nano-DDS-based tumor-targeted diagnostic contrast agent may be used in fluorescence-guided surgery and/or a biopsy. Also, the contrast agent may be capable of repeatedly penetrating and being absorbed into the tumor in a repetitive tumor removal process. Specifically, when penetration/absorption of the contrast agent and tumor removal are set as one cycle, 2 cycles or more, preferably, 2 to 10 cycles, 2 to 6 cycles, or 3 to 5 cycles may be performed. In an embodiment of the present invention, penetration/absorption of the contrast agent and tumor removal may be each performed three times, and it was confirmed that the absorption/penetration efficiency of the contrast agent was excellent all three times.

In the present invention, the "first mixture formation step" is a step of mixing a polysorbate, soybean oil, and a staining substance to form a first mixture.

In an embodiment, the polysorbate is a component used as a surfactant in medicines or cosmetics due to having both hydrophilicity and hydrophobicity. The polysorbate also has characteristics as a stabilizer that helps particles to be stably dispersed in an emulsification process or a solubilizing agent that allows a water-insoluble active ingredient to be dissolved.

In an embodiment, the polysorbate may be selected from polysorbate 20, polysorbate 80, and the like

In an embodiment, the soybean oil is a water-insoluble component that does not mix with water and is loaded in particles while forming a hydrophobic bond with a water-insoluble active ingredient in an emulsification process. In the present invention, the staining substance can be loaded in the nanoplatform using the soybean oil.

The soybean oil may be included in an amount of 1 to 60 parts by weight, 5 to 50 parts by weight, or 10 to 30 parts by weight relative to 100 parts by weight of the polysorbate.

In an embodiment, the staining substance is a water-insoluble staining substance that is able to emit light by a light source with a wavelength of 650 to 800 nm, 700 to 800 nm, or 750 to 800 nm. The staining substance is able to penetrate/be absorbed into tissue and may be applied in clinical practice and operating rooms.

There is no particular limitation on the type of staining substance, and the staining substance may be one or more selected from the group consisting of cyanine dyes, chlorin e6, and methylene blue. Examples of the cyanine dyes include Cy5.5, Cy7, IR-780, IR-783, IR-808, indocyanine green, and the like.

In addition, the staining substance may be included in an amount of 0.01 to 1 part by weight or 0.1 to 0.5 parts by weight relative to 100 parts by weight of the polysorbate. Within the above content range, the staining substance can be stably dispersed and easily loaded in the nano-DDS. The above mixing ratio is an example of a mixing ratio capable of exhibiting the above-described effects intended by the present invention.

In an embodiment, a first mixture may be prepared in the form of a solution by mixing the above-described components, that is, the polysorbate, soybean oil, and staining substance. The first mixture is transparent and uniform without a foreign body sensation.

In the present invention, the "second mixture formation step" is a step of mixing the first mixture with a poloxamer and a stabilizer and heating the resulting mixture at 40 to 70 °C to form a second mixture.

In the present invention, the poloxamer is a triblock copolymer in which a hydrophilic group and a hydrophobic group are bonded. The poloxamer may have a structure of poly(oxyethylene)-poly(oxypropylene)-poly(oxyethylene) [POE-POP-POE]. The poloxamer is solid at room temperature and is soluble in water and ethanol, and a poloxamer solution is liquid at low temperature, but the viscosity thereof is increased when a temperature is increased, and the poloxamer solution exhibits the thixotropy of sol-gel depending on a temperature when a concentration is high. Also, the poloxamer does not damage mucosal cells.

As the poloxamer, commercially available poloxamer 188 and poloxamer 407 may be used. Poloxamer 188 and poloxamer 407 may be used in clinical practice. In the present invention, poloxamer 188 may be used as the poloxamer. For example, poloxamer 188 refers to a compound having a structure of (POE)ₐ-(POP)_{b}-(POE)_{c} in which b is 30 and the sum of a and c is about 75 and a molecular weight of about 8350.

In general, the introduction of a hydrophilic group is intended to impart functionality so that a substance and an active ingredient are uniformly and stably dispersed in an aqueous solution for a long time while not being aggregated, and is also essential to impart an important function of delivering an active ingredient to a desired disease site by increasing body circulation when administered to a blood vessel. However, in the present invention, a hydrophilic group is introduced so that penetration and absorption into cells or tissue is possible, and the introduction is intended to impart a function of stably and uniformly dispersing ultrafine nano-sized (8 nm to 10 nm) particles in an aqueous solution. In addition, the introduction of a hydrophobic group is an essential element for loading a water-insoluble active ingredient (various drugs or staining substances) having the same physical properties in the inner core of a nanoplatform.

In the present invention, the stabilizer may be used to improve the stability of the nano-DDS-based tumor-targeted diagnostic contrast agent loaded with the staining substance in a hydrated state and the light stability of the staining substance.

In an embodiment, the stabilizer may be one or more selected from the group consisting of Solutol and vitamin E TPGS.

Solutol, which is an amphoteric polymer having hydrophilicity and hydrophobicity, is a substance used as a non-ionic solubilizing agent or an emulsifier and consists of 15 moles of ethylene oxide (main component of polyethylene oxide (PEG)) and 1 mole of 12-hydroxystearic acid. Since the main chain thereof has a lipophilic stearic structure, it can exhibit excellent miscibility with a hydrophobic drug.

As the Solutol, Solutol HS-15 may be used.

In addition, vitamin E TPGS is a vitamin E derivative in which a polyethylene glycol subunit is attached to the ring hydroxyl of a vitamin E molecule by succinate diester. Vitamin E TPGS stands for D-α-tocopherol polyethylene glycol 1000 succinate (MW=530). In this case, TPGS is a non-ionic surfactant.

In an embodiment, the stabilizer may be included in an amount of 10 to 90 parts by weight or 30 to 80 parts by weight relative to 100 parts by weight of the poloxamer. Also, the poloxamer and the stabilizer may be included in an amount of 5 to 50 parts by weight or 10 to 30 parts by weight relative to the total weight of the first mixture. Within the above content range, a nano-DDS can be stably prepared.

In an embodiment, a heating temperature may be 40 to 70 °C or 50 to 60 °C. When the heating temperature is less than 40 °C, melting is not performed due to the heating temperature being lower than the melting point of the poloxamer, and thus mixing with the first mixture may not be performed, and when the heating temperature exceeds 70 °C, the poloxamer and the active ingredient may be decomposed.

In an embodiment, a second mixture is prepared by adding the poloxamer and the stabilizer to the solution obtained by mixing the above-described components, that is, the polysorbate, soybean oil, and staining substance, and may also be transparent and uniform.

In the present invention, the "nanoplatform solid formation step" is a step of cooling the second mixture to form a nanoplatform solid.

In an embodiment, a cooling temperature may be 15 to 30 °C or 15 to 25 °C. When the second mixture is cooled after being prepared by reaction at a temperature above the melting point of a polymer, that is, the poloxamer, the mixture is solidified, and in this process, the poloxamer may surround the staining substance to load the staining substance inside the particle. When the cooling temperature exceeds 30 °C, the crystallization of particles is decreased as the second mixture is slowly cooled, and thus the uniformity of a particle size is decreased. In the present invention, since the cooling temperature is set to 15 to 30 °C, a nano-sized nanoplatform solid can be easily prepared without using a medium for rapid cooling.

In the present invention, a nanoplatform solid may be formed by this step. Nano may refer to a size of 1 to 1,000 nm. The nanoplatform may be a carrier or vehicle that is able to load the above-described staining substance. Also, the nanoplatform solid may include a carrier for a drug or contrast agent, which is applicable in the technical field to which the present invention belongs.

In addition, the method of the present invention may further include:
mixing the nanoplatform solid with a solvent to form a third mixture (hereinafter, referred to as a third mixture formation step);
removing impurities from the third mixture (hereinafter, referred to as an impurity removal step); and
freeze-drying the third mixture from which impurities are removed (hereinafter, referred to as a freeze-drying step).

In the present invention, the "third mixture formation step" is a step of mixing the nanoplatform solid with a solvent to form a third mixture.

In an embodiment, the solvent may be tertiary distilled water. The nanoplatform solid may be dissolved in the solvent, and the active ingredient or other substances included along with the active ingredient may be uniformly and stably dispersed without a foreign body sensation caused by aggregation.

The present invention is characterized in that an organic solvent is not used. When an organic solvent is used, there is a concern that the form of an agent loaded with an active ingredient may disintegrate, and thus become unusable. Also, even when disintegration does not occur, a process of removing an organic solvent needs to be included, and there is the possibility of inducing toxicity in the body when the used solvent remains.

In an embodiment, since there is no possibility of inducing toxicity due to the solvent in the preparation process when tertiary distilled water is used as the solvent, the method of the present invention is differentiated from a conventional method (self-assembly emulsification) using an organic solvent and commonly used to prepare a liposome or a polymer-based nanoplatform by having high biocompatibility.

In an embodiment, a third mixture may be formed by this step. The third mixture may be transparent and uniform.

In the present invention, the "impurities removal step" is a step of removing impurities from the third mixture.

In an embodiment, an impurity removal method is not particularly limited, and any method applicable to the technical field to which the present invention belongs may be used. For example, impurities may be removed using a 0.2 to 1.2 µm filter, a 0.5 to 1.1 µm filter, a 0.6 to 1.0 µm filter, a 0.7 to 0.9 µm filter, or a 0.8 µm filter.

In the present invention, the "freeze-drying step" is a step of freeze-drying the third mixture from which impurities are removed.

In an embodiment, the freeze-drying is a moisture removal method and is a method of freezing an object and then lowering atmospheric pressure so that solid-state water sublimates to a gas state. Although the freeze-drying method is not specifically presented, it is obvious that any freeze-drying method applicable to the technical field to which the present invention belongs may be applied.

In an embodiment, a solid-type nanocomposite may be formed by this step. The nanocomposite has a unique color of the active ingredient and maintains unique spectroscopic properties.

In addition, the present invention relates to a nano-DDS-based tumor-targeted diagnostic contrast agent prepared by the above-described method of preparing a nano-DDS-based tumor-targeted diagnostic contrast agent.

A nano-DDS-based tumor-targeted diagnostic contrast agent according to the present invention is a nanocomposite with a micelle structure, wherein an inner core of the micelle structure includes soybean oil and a polysorbate in which a staining substance is dissolved, and an outer shell of the micelle structure includes a poloxamer and a stabilizer.

In this case, the stabilizer may be one or more selected from the group consisting of Solutol and vitamin E TPGS.

In an embodiment, the nano-DDS-based tumor-targeted diagnostic contrast agent may have an average diameter of 1 to 500 nm, 1 to 100 nm, 1 to 90 nm, 1 to 80 nm, 1 to 70 nm, 1 to 60 nm, 1 to 50 nm, 1 to 40 nm, 1 to 30 nm, 1 to 20 nm, 3 to 15 nm, 5 to 12 nm, 7 to 10 nm, or 8 to 9 nm. When the average diameter of the nano-DDS-based tumor-targeted diagnostic contrast agent is excessively large, direct absorption into tissue such as a tumor and the like is difficult, and thus the contrast agent may not be applied to the application method intended by the present invention. In other words, as described above, the present invention can provide a nanoplatform optimized for a direct application method, which could not be implemented by conventional nano-sized carrier described in some related art literature, by controlling an average diameter of the nano-DDS-based tumor-targeted diagnostic contrast agent to an ultrafine scale.

When the nano-DDS-based tumor-targeted diagnostic contrast agent does not have a nano size, the rate of penetration and absorption into tissues is not high. For this reason, it is important for the contrast agent to have an average diameter in the above-described range. Due to the above-described size, a larger amount of the contrast agent is absorbed into and penetrates tumor tissue compared to normal tissue, and thus the tumor may be distinguished.

In an embodiment, the nano-DDS-based tumor-targeted diagnostic contrast agent may be used as a diagnostic agent capable of selectively staining abnormal inflammatory tissue or tumor tissue. Specifically, the nano-DDS-based tumor-targeted diagnostic contrast agent may be used in fluorescence-guided surgery and/or a biopsy and may repeatedly penetrate the tumor in a repetitive tumor removal process.

In addition, in an embodiment, the staining substance loaded in the nano-DDS-based tumor-targeted diagnostic contrast agent has a water-insoluble property, and the water-insoluble staining substance is hardly dissolved unless an organic solvent such as ethanol, methanol, acetone, DMSO, or the like is used. Also, the staining substance is non-uniform because it does not mix at all in tertiary distilled water and either floats or settles.

Additionally, a contrast agent solution in which the above-described nano-DDS-based tumor-targeted diagnostic contrast agent of the present invention is dispersed in an injection solution or a buffer solution is provided. The contrast agent solution is uniform and transparent.

Therefore, the nano-DDS-based contrast agent that is selectively delivered only to a tumor or abnormal tissue transformed by inflammation to stain the tumor or abnormal tissue may be provided.

In addition, when the nano-DDS-based tumor-targeted diagnostic contrast agent is applied throughout regardless of the specific area where a disease occurs, and washed with normal saline, selective staining is possible due to high penetration and absorption rates of the labeling agent into abnormal tissue or tumor tissue transformed by inflammation as compared to normal tissue. Therefore, precise staining (diagnosis) of the diseased area is possible.

Additionally, repeated precise staining (diagnosis) of the tumor in a repetitive tumor removal process is possible.

In an embodiment, normal tissue and tumor tissue may be precisely distinguished by uniformly dispersing the nano-DDS-based tumor-targeted diagnostic contrast agent in a solution (injection solution, buffer solution, distilled water, etc.) suitable for the purpose and then administering the contrast agent solution via a blood vessel or directly applying the same to tumor tissue.

In an embodiment, as the injection solution, a 0.5% to 1.3% NaCl solution, a 0.6% to 1.2% NaCl solution, a 0.7% to 1.1% NaCl solution, a 0.8% to 1.0% NaCl solution, or a 0.9% NaCl solution may be used. Also, a 2% to 8% glucose solution, a 3% to 7% glucose solution, a 4% to 6% glucose solution, or a 5% glucose solution may be used.

Hereinafter, the present invention will be described in detail by way of examples. However, the following examples are merely presented to exemplify the present invention, and the content of the present invention is not limited to the following examples. The examples of the present invention serve to complete the disclosure of the present invention, and are provided to convey the full scope of the invention to those skilled in the art to which the present invention pertains. The present invention should be defined based on the scope of the appended claims.

### Examples

### Example 1. Preparation of nanoplatform loaded with staining substance for cancer diagnosis

Using thermal phase transition of a polymer, a nano-DDS-based tumor-targeted diagnostic contrast agent loaded with a physicochemically water-insoluble staining substance for cancer diagnosis was prepared.

0.01 g of a staining substance for cancer diagnosis (a staining substance that emits light at a wavelength of 700 to 800 nm) was input into a reaction container, 0.6 g of soybean oil and 3 g of Tween 80 were added as solubilizing agents, and then stirring was sufficiently performed at room temperature so that the resulting mixture was transparent and uniform. Afterward, 0.4 g of Kolliphor P188 and 0.3 g of vitamin E TPGS as a stabilizer for improving the stability of particles loaded with the staining substance in a hydrated state and the light stability of the staining substance were added, heated at 50 to 55 °C, and mixed by sufficient stirring to prepare a transparent, uniform, and slightly viscous liquid. Then, the resulting liquid was cooled to room temperature (about 24 °C) to prepare a primary solid. Also, the staining substance that was not loaded in the nano-DDS platform but aggregated was removed.

The obtained primary solid was completely dispersed in tertiary distilled water (18.2 mΩ or more), sterilized by filtration with a 0.2 µm filter, and freeze-dried to finally obtain a nano-DDS-based tumor-targeted diagnostic contrast agent.

The absorption/penetration mechanism of the nano-DDS-based tumor-targeted diagnostic contrast agent according to the present invention into tissue is shown in FIG 1.

### Comparative Example 1. Preparation of nanoplatform not including stabilizer

A nano-DDS-based tumor-targeted diagnostic contrast agent was prepared in the same manner as in Example 1, except that a stabilizer was not used.

### Experimental Example 1. Evaluation of stability of nano-DDS-based tumor-targeted diagnostic contrast agent in aqueous solution

The nano-DDS-based tumor-targeted diagnostic contrast agent is present in a dry state and may have a nano size when dispersed in an aqueous solution. When the contrast agent is dispersed in an aqueous solution, precipitation (aggregation) of the staining substance in the contrast agent may hinder accurate cancer diagnosis, and therefore, the precipitation needs to be minimized. In other words, when stably maintained in a hydrated state in the nano-DDS for a predetermined time without being precipitated, the staining substance may sufficiently reach the inside of desired cancer tissue.

In addition, when light stability is ensured according to physicochemical properties of the staining substance that emits light by a light source having a specific external wavelength (λmax=780 to 800 nm), diagnostic efficiency may be improved.

Therefore, in this experimental example, the stability of the nano-DDS-based tumor-targeted diagnostic contrast agent in an aqueous solution was evaluated.

First, each nano-DDS-based tumor-targeted diagnostic contrast agent prepared in Example 1 and Comparative Example 1 was dispersed in a 0.9% NaCl injection solution, and then a change in aqueous solution state was observed over time.

In addition, to confirm light stability, the aqueous solution including the nano-DDS-based tumor-targeted diagnostic contrast agent dispersed therein was directly irradiated with a light source having a specific wavelength, and then a change in luminescence intensity were observed over time through UV-vis spectroscopy.

Results thereof are shown in FIG 2.

FIG 2A shows the particle distribution of the nano-DDS-based tumor-targeted diagnostic contrast agent prepared in Example 1 in an aqueous solution, FIG 2B shows a change of the diagnostic contrast agent in an aqueous solution, and FIG 2C shows light stability by an external light source.

In this case, the contrast agent prepared in Comparative Example 1 was used as a "conventional nano-DDS contrast agent," and the contrast agent prepared in Example 1 was used as a "nano-DDS contrast agent with improved stability."

The particle distribution and change of the diagnostic contrast agent in an aqueous solution were measured using a particle size analyzer, and light stability was measured through UV-vis spectroscopy.

As shown in FIG 2A, it can be confirmed that the nano-DDS-based tumor-targeted diagnostic contrast agent prepared in Example 1 was easily dissolved in an aqueous solution and exhibited an average particle size of about 10 nm. Also, as shown in FIG 2B, it can be confirmed that, since a stabilizer was used for stability improvement, the contrast agent of Example 1 was maintained in a dispersed state under a hydrated condition for a predetermined time, as compared to the contrast agent of Comparative Example 1.

In addition, as shown in FIG 2C, it can be confirmed that, when a stabilizer was used, a rapid decrease in light stability was delayed upon exposure to light, and thus light stability by an external light source was also supplemented. Cancer may be classified according to luminescence intensity in cancer diagnosis, and as light stability decreases, luminescence intensity also decreases, and thus it may be difficult to clearly classify cancer. Therefore, it is important to ensure light stability.

### Experimental Example 2. Evaluation of penetration/absorption time of nano-DDS-based tumor-targeted diagnostic contrast agent into tumor tissue in inflammatory colorectal cancer (AOM/DSS) mouse model

Since the nano-DDS-based tumor-targeted diagnostic contrast agent must be used during surgical cancer removal in an operating room, it is necessary to stain desired cancer tissue as quickly as possible and provide diagnostic information to the surgeon. Therefore, although at a non-clinical level, the nano-DDS-based tumor-targeted diagnostic contrast agent prepared in Example 1 (dispersed in a 0.9% NaCl injection solution) was applied to an inflammatory colorectal cancer-induced model, and then diagnosis and the degree of diagnosis were confirmed over time.

Results thereof are shown in FIG 3.

FIG 3 show results of observing a colorectal cancer-induced mouse model over time using real-time near-infrared fluorescence diagnostic equipment for small animals after directly spraying the nano-DDS-based tumor-targeted diagnostic contrast agent prepared in Example 1 onto the mouse model.

As shown in FIG 3, it can be confirmed that the nano-DDS-based tumor-targeted diagnostic contrast agent enabled tumor diagnosis even when applied to a desired site for a short period of time.

### Experimental Example 3. Under assumption of surgical cancer removal situation, evaluation of diagnosis and tumor tissue extraction according to repeated application of nano-DDS-based tumor-targeted diagnostic contrast agent to tumor formed in tumor-induced mouse model

When unremoved tumor tissue remains after a clinician diagnoses and then extracts a tumor in an operating room, there is a high likelihood of recurrence. Therefore, in order to completely remove the residual tumor cells, whether re-diagnosis is possible according to repeated application is very important.

For this purpose, a process of directly applying the nano-DDS-based tumor-targeted diagnostic contrast agent prepared in Example 1 to the tumor of a tumor-induced mouse model and performing washing to primarily remove tumor tissue, and additionally performing application and washing to remove the residual tumor tissue was repeatedly performed.

Results thereof are shown in FIG 4.

FIG 4 shows images obtained by measurement according to a repeated process using near-infrared fluorescence imaging equipment after a process of spraying the nano-DDS-based tumor-targeted diagnostic contrast agent prepared in Example 1 onto the multiple tumors of a tumor-induced mouse model, performing diagnosis, and extracting cancer tissue is repeatedly performed. This assumes the situation of an operating room where diagnosis and tissue extraction are repeatedly performed.

As shown in FIG 4, it can be confirmed that the nano-DDS-based tumor-targeted diagnostic contrast agent according to the present invention enabled diagnosis by penetrating/being absorbed into the suspected tissue even when repeatedly applied onto the same site after primary tumor removal. From this result, it can be seen that the residual tumor tissue can be additionally removed after primary tumor removal.

### Experimental Example 4. Evaluation of diagnostic potential of nano-DDS-based tumor-targeted diagnostic contrast agent according to type of cancer formed in inflammatory colorectal cancer mouse model

FIG 5A is a schematic diagram showing various histological stages of the progression of colorectal cancer.

Since tumors rapidly grow from cells to tissues, there is a difference according to the initial site of occurrence. Therefore, when tumors are detected in early diagnosis, treatment can be quickly started. However, tumors originating from the inside of tissue are likely to be missed in general cancer screening not using CT, MRI, or PET, which are high-tech methods for radiology, and when there are no special initial subjective symptoms, the tumor is found in a considerably advanced state, and thus treatment is difficult. Therefore, when diagnosis of not only tumors formed at the tissue surface but also subepithelial tumors (or submucosal tumors) formed under the epithelial layer is possible, the value and potential of a contrast agent are greatly increased.

For this purpose, the nano-DDS-based tumor-targeted diagnostic contrast agent prepared in Example 1 of the present invention was directly sprayed onto the multiple tumors of an inflammatory colorectal cancer-induced mouse model and washed, and then measurement was performed using near-infrared fluorescence imaging equipment.

FIG 5B shows images obtained by measurement using near-infrared fluorescence imaging equipment.

As shown in FIG 5B, it can be confirmed that cancer was stained, and thus diagnosis is possible.

In addition, the inflammatory colorectal cancer-induced mouse model onto which the nano-DDS-based tumor-targeted diagnostic contrast agent was sprayed was subjected to a frozen section biopsy for tissue. Afterward, hematoxylin and eosin (H&E) staining, which is widely used in medical diagnosis and the most common tissue staining method, was performed, then observation was performed using a general optical microscope for tissue, and comparative analysis was performed using a fluorescence microscope for tissue.

FIGS. 6A and 6B show images obtained by measuring the tissue stained with H&E using a microscope and images obtained by measuring the same tissue section using a fluorescence microscope, respectively, after the nano-DDS-based tumor-targeted diagnostic contrast agent prepared in Example 1 was directly sprayed onto the multiple tumors of an inflammatory colorectal cancer-induced mouse model and washed, and the tissue whose cancer diagnosis was determined was sliced into the form of a section.

As shown in FIG 6, it can be confirmed that the nano-DDS-based tumor-targeted diagnostic contrast agent enabled diagnosis by penetrating/being absorbed into not only cancer formed at the surface but also subepithelial tumors that are difficult to identify without detailed examination.

In addition, a surgeon needs to perform a tissue biopsy at the end to confirm that cancer has been completely removed through the above series of processes. Using this, a pathologist can quickly confirm and analyze the presence of histopathological tumor cells in histopathological examination, so it can be sufficiently utilized in related fields.

### Experimental Example 5. Evaluation of penetration/absorption mechanism of nano-DDS-based tumor-targeted diagnostic contrast agent into tumor tissue

The absorption mechanism of the nano-DDS-based tumor-targeted diagnostic contrast agent into tumor tissue in three-dimensionally cultured cells was confirmed.

First, 5 × 10⁴ cells/well of small intestinal mucosal cells (Caco-2 cells) were three-dimensionally cultured using trans-wells (BD Biosciences) of a 24-well plate, treatment with the nano-DDS-based tumor-targeted diagnostic contrast agent prepared in Example 1 was performed for a predetermined time, washing was performed, and then the absorption mechanism was confirmed.

Results thereof are shown in FIG 7.

FIG 7 shows images and fluorescence quantitative analysis results obtained by measurement using a fluorescence microscope after the nano-DDS-based tumor-targeted diagnostic contrast agent prepared in Example 1 was applied to three-dimensionally cultured cells and washed.

As shown in FIG 7, it can be confirmed that only a green actin-stained image was observed in the control group (nano-contrast agent X) treated with nothing, whereas intense red fluorescence in the form of a mesh was expressed in the experimental group (nano-contrast agent O) treated with the nano-DDS-based tumor-targeted diagnostic contrast agent, and there is also a difference in fluorescence quantitative analysis.

When the stained image using an FITC-phalloidin marker, which is able to stain the action of tight junction in green, and the stained image using the nano-DDS-based tumor-targeted diagnostic contrast agent are overlapped, the two images match, and therefore, it can be confirmed that the nano-DDS-based tumor-targeted diagnostic contrast agent according to the present invention can be absorbed orally through the tight junction.

In the present invention, a nano-DDS-based tumor-targeted diagnostic contrast agent can be prepared by a simple method based on thermal phase transition of a polymer without using anhydrous ethanol, a non-polar organic solvent, or an aqueous solution.

A fluorescent substance (staining substance) loaded in the nano-DDS-based tumor-targeted diagnostic contrast agent according to the present invention can have high particle stability in an aqueous solution.

In the present invention, tumors can be effectively diagnosed by applying the nano-DDS-based tumor-targeted diagnostic contrast agent to the local area through simple spraying and then performing washing. Also, tumors can be effectively diagnosed and repeatedly removed by a repeated process of applying the contrast agent to the local area through simple spraying and performing washing.

In addition, since the nano-DDS-based tumor-targeted diagnostic contrast agent according to the present invention has a high delivery effect into cells, tumors formed in not only the mucosal layer but also the submucosal layer can also be diagnosed and removed.

## Claims

1. A method of preparing a nano-drug delivery system (DDS)-based tumor-targeted diagnostic contrast agent, the method comprising:
mixing a polysorbate, soybean oil, and a staining substance to form a first mixture;
mixing the first mixture with a poloxamer and a stabilizer and heating the resulting mixture at 40 to 70 °C to form a second mixture; and
cooling the second mixture to form a nanoplatform solid,
wherein the staining substance is a staining substance that emits light at a wavelength of 650 to 800 nm, and
the stabilizer is one or more selected from the group consisting of Solutol and vitamin E TPGS.

2. The method of claim 1, wherein the nano-DDS-based tumor-targeted diagnostic contrast agent is used in fluorescence-guided surgery and/or a biopsy and is able to repeatedly penetrate and be absorbed into a tumor in a repetitive tumor removal process.

3. The method of claim 1, wherein the staining substance is one or more selected from the group consisting of cyanine dyes, chlorin e6, and methylene blue.

4. The method of claim 1, wherein the soybean oil is included in an amount of 1 to 60 parts by weight relative to 100 parts by weight of the polysorbate.

5. The method of claim 1, wherein the staining substance is included in an amount of 0.01 to 1 part by weight relative to 100 parts by weight of the polysorbate.

6. The method of claim 1, wherein the stabilizer is mixed in an amount of 10 to 90 parts by weight relative to 100 parts by weight of the poloxamer.

7. The method of claim 1, wherein the poloxamer and the stabilizer are included in an amount of 5 to 50 parts by weight relative to the total weight of the first mixture.

8. The method of claim 1, further comprising:
mixing the nanoplatform solid with a solvent to form a third mixture;
removing impurities from the third mixture; and
freeze-drying the third mixture from which impurities are removed.

9. A nano-DDS-based tumor-targeted diagnostic contrast agent which is a nanocomposite with a micelle structure,
wherein an inner core of the micelle structure includes soybean oil and a polysorbate in which a staining substance is dissolved, and
an outer shell of the micelle structure includes a poloxamer and a stabilizer.

10. The nano-DDS-based tumor-targeted diagnostic contrast agent of claim 9, wherein the nano-DDS-based tumor-targeted diagnostic contrast agent has a size of 1 to 500 nm.
